# EUROPÄISCHE PATENTSCHRIFT

(11) **EP 0 545 966 B1**
(45) Veröffentlichungstag und Bekanntmachung des Hinweises auf die Patenterteilung: **23.11.1994**
(21) Anmeldenummer: 91914553.2
(22) Anmeldetag: 14.08.1991
(51) Int. Cl.: C07H 19/10, C07H 19/20, A61K 31/70, C12P 19/28

(54) **NEUE PHOSPHOLIPID-DERIVATE VON NUCLEOSIDEN, DEREN HERSTELLUNG SOWIE DEREN VERWENDUNG ALS ANTIVIRALE ARZNEIMITTEL**
NEW PHOSPHOLIPID DERIVATIVES OF NUCLEOSIDES, THEIR PREPARATION AND THEIR USE AS ANTIVIRAL DRUGS
NOUVEAUX DERIVES PHOSPHOLIPIDIQUES DE NUCLEOSIDES, LEUR PRODUCTION ET LEUR UTILISATION COMME MEDICAMENTS ANTIVIRAUX

(30) Priorität: 20.08.1990 DE 4026265
(43) Veröffentlichungstag der Anmeldung: 16.06.1993
(73) Patentinhaber: BOEHRINGER MANNHEIM GMBH, 68298 Mannheim (DE)
(72) Erfinder: ZILCH, Harald, D-6800 Mannheim 31 (DE); LEINERT, Herbert, D-6148 Heppenheim (DE); MERTENS, Alfred, D-6905 Schriesheim (DE); HERRMANN, Dieter, D-6900 Heidelberg (DE)
(74) Vertreter: Weber, Manfred, Dr.
(86) Internationale Anmeldenummer: EP9101541
(87) Internationale Veröffentlichungsnummer: WO9203462

(56) Entgegenhaltungen:
- The Journal of Biological Chemistry, vol. 265, No.11, 15 Apr. 1990. American Society for Biochemistry and Molecular Biology, (US), K.Y. Hostetler et al.: "Synthesis and antiretroviral activity of phospholipid analogs of azidothymidine and other antiviral nucleosides" pages 6112-6115. see the whole document (cited in the application)
- Tetrahedron Lettes, vol. 28, No. 1. 1987, Pergamon Press (GB), S. Shuto et Al.; "A facile one-step synthesis of 5'-phosphatidylnucleosides by an enzymatic two-phase reaction" pages 199-202. see abstract (cited in te application)
- Journal of Medicinal Chemistry, vol. 33, No.5, May 1990, American Chemical Society, C II Hong et al.: "Nucleoside conjugates.11 Synthesis and antitumor activity of 1-beta-D-arabinofuranosylcytosine and cytidine conjugates of thioether lipids", pages 1380-1386, see whole document (cited in the application)
- Chemical and Pharmaceutical Bulletin, volume36, No. 12, December 1988, Pharmaceutical Society of Japan (JP), S. Shuto et al.:"Phospholipase D-catalyzed trans-alkylphosphorylation: a facile one-step synthesis of nucleoside 5'-alkylphosphates", pages 5020-5023, see abstract; pages 5021 (cited in the application)
- Chemical and Pharmaceutical Bulletin, vol.36, No. 1, January 1988, Pharmaceutical Society of Japan,(JP), S. Shuto et al.: "A facile enzymatic synthesis of 5'-(3-sn-phosphatidyl) nucleosides and their antileukemic activities", pages 209-217, see abstract; card 1 (cited in the application)
- Journal of Medicinal Chemistry, vol. 34, No. 4, Apr. 1991, American Chemical Society, C. Piantadosi at al.: "Synthesis and evaluation of novel ether lipid nucleoside conjugates for anti-HIV-1 activity"pages 1408-1414, see the whole document

## Beschreibung

Gegenstand der vorliegenden Erfindung sind neue Phospholipid-Derivate von Nucleosiden der allgemeinen Formel I, 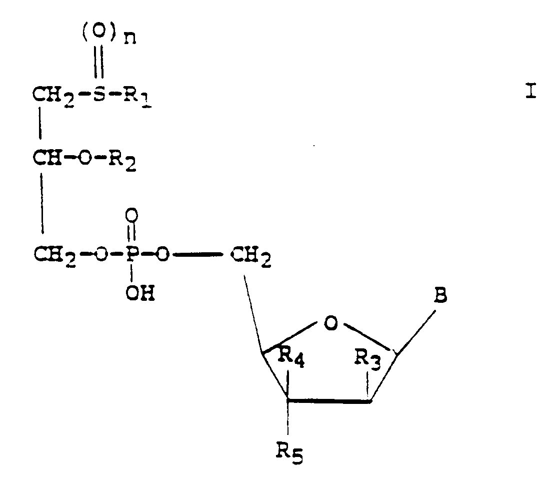
in der
- R₁: eine geradkettige oder verzweigte, gesättigte oder ungesättigte Alkylkette mit 8-15 Kohlenstoffatomen, die gegebenenfalls ein oder mehrfach durch Phenyl-, Halogen, C₁-C₆-Alkoxy-, C₁-C₆-Alkylmercapto-, C₁-C₆-Alkoxycarbonyl-, C₁-C₆-Alkylsulfinyl- oder C₁-C₆-Alkylsulfonylgruppen substituiert sein kann,
- R₂: eine geradkettige oder verzweigte, gesättigte oder ungesättigte Alkylkette mit 8-15 Kohlenstoffatomen, die gegebenenfalls ein oder mehrfach durch Phenyl-, Halogen, C₁-C₆-Alkoxy-, C₁-C₆-Alkylmercapto-, C₁-C₆-Alkoxycarbonyl- oder C₁-C₆-Alkylsulfonylgruppen substituiert sein kann,
- R₃: Wasserstoff oder eine Hydroxygruppe,
- R₄, R₅: jeweils Wasserstoff oder einer der Reste R₄ und R₅ Halogen, eine Hydroxy-, eine Cyano- oder eine Azidogruppe bedeuten und außerdem R₃ und R₄ eine weitere Bindung zwischen C-2' und C-3' darstellen können,
- n: 0, 1 oder 2 und
- B: eine der folgenden Verbindungen bedeutet:

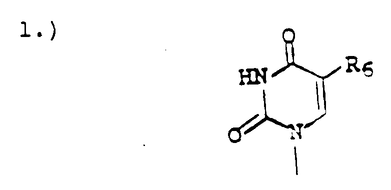

wobei R₆ Wasserstoff, eine Alkylkette mit 1-4 Kohlenstoffatomen oder Halogen sein kann, 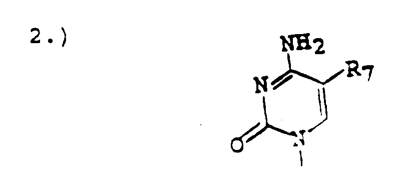
wobei R₇ Wasserstoff, eine Alkylkette mit 1-4 Kohlenstoffatomen oder Halogen sein kann, 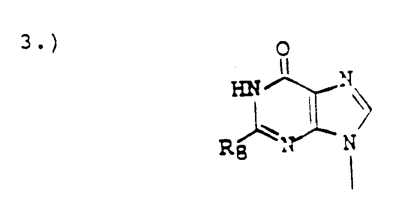
wobei R₈ Wasserstoff, eine Alkylkette mit 1-4 Kohlenstoffatomen, Halogen, oder eine Hydroxy- oder eine Aminogruppe sein kann, 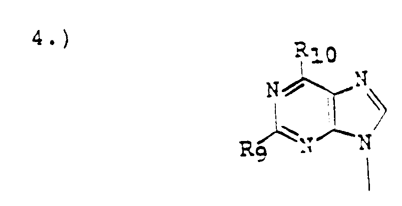
wobei R₉ Wasserstoff oder eine Aminogruppe sein kann, und R₁₀ Wasserstoff, Halogen, C₁-C₆-Alkoxy, C₁-C₆-Alkylmercapto, oder eine Aminogruppe, die mono- oder disubstituiert sein kann durch C₁-C₆-Alkyl-, C₁-C₆-Alkoxy-, Hydroxy-C₂-C₆-alkyl- und/oder C₃-C₆-Cycloalkyl-, Aryl-, Hetaryl-, Aralkyl- oder Hetarylalkylgruppen, die gegebenenfalls im Aryl- oder Hetarylrest noch durch eine oder mehrere Hydroxy-, Methoxy- oder Alkylgruppen oder Halogen substituiert sein können, oder Allyl, das gegebenenfalls mit Mono- oder Dialkyl- oder Alkoxygruppen substituiert sein kann, bedeutet,
deren Tautomere und deren physiologisch verträgliche Salze anorganischer und organischer Säuren bzw. Basen, sowie Verfahren zu ihrer Herstellung und diese Verbindungen enthaltende Arzneimittel.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

In J. Biol. Chem. 265, 6112 (1990) ist die Herstellung und Verwendung von Liponucleotiden als antivirale Arzneimittel beschrieben. Untersucht und synthetisiert wurden hier aber nur die an bekannte Nucleoside, wie z.B. AZT und ddC, gekoppelten Dimyristoylphosphatidyl- und Dipalmitoylphosphatidylreste mit ihrer Fettsäureesterstruktur.

In J. Med. Chem. 33, 1380 (1990) sind Nucleosid-Konjugate von Thioetherlipiden mit Cytidindiphosphat beschrieben, die eine antitumorale Wirkung aufweisen und Verwendung in der Onkologie finden könnten.

In Chem. Pharm. Bull. 36, 209 (1988) sind 5'-(3-SN-Phosphatidyl)nucleoside mit antileukämischer Aktivität beschrieben sowie deren enzymatische Synthese aus den entsprechenden Nucleosiden und Phosphocholinen in Gegenwart von Phospholipase D mit Transferaseaktivität.

Die enzymatische Synthese von Liponucleotiden ist u.a. ebenfalls in Tetrahedron Lett. 28, 199 (1987) und Chem. Pharm. Bull. 36, 5020 (1988) beschrieben.

Die Verbindungen der vorliegenden Erfindung weisen ebenfalls wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papova-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und HIV-2, verursacht werden.

Besonders geeignet erscheinen die Verbindungen der Formel I zur Behandlung der klinischen Manifestationen der retroviralen HIV-Infektion beim Menschen, wie der anhaltenden generalisierten Lymphadenopathie (PGL), dem fortgeschrittenen Stadium des AIDS-verwandten Komplex (ARC) und dem klinischen Vollbild von AIDS.

Überraschenderweise wurde nun gefunden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987). Von besonderem therapeutischem Interesse ist die Hemmwirkung auf das HIV, dem Verursacher der Immunschwäche-Erkrankung AIDS. Zur Behandlung von AIDS ist heute nur 3'-Azido-3'desoxythymidin (DE-A-3608606) bei AIDS Patienten zugelassen. Jedoch machen toxische Nebenwirkungen des 3'-Azido-3'-desoxythymidins auf das Knochenmark bei etwa 50 % der behandelten Patienten Bluttransfusionen erforderlich. Die Verbindungen der allgemeinen Formel I besitzten diese Nachteile nicht. Sie wirken antiviral, ohne in pharmakologisch relevanten Dosen cytotoxisch zu sein.

Die Verbindungen der vorliegenden Erfindung und ihre pharmazeutischen Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind wie 3'-Azido-3'-desoxythymidin, 2',3'-Didesoxynucleoside wie z. B. 2',3'-Didesoxycytidin, 2',3'-Didesoxyadenosin und 2',3'-Didesoxyinosin, acyclische Nucleoside (z. B. Acyclovir), Interferone wie z. B. A-Interferon, renale Ausscheidungs-Inhibitoren wie. z. B. Probenicid, Nucleosid-Transport-Inhibitoren wie z. B. Dipyridamol, als auch Immunmodulatoren wie z. B. Interleukin II oder Stimulierungs-Faktoren wie z. B. der Granulocyten-Makrophagen-Kolonie Faktor. Die Verbindungen der vorliegenden Erfindung und das andere Arzneimittel können jeweils einzeln, gleichzeitig gegebenenfalls in einer einzigen oder zwei getrennten Formulierungen oder zu unterschiedlichen Zeiten verabreicht werden, so daß ein synergistischer Effekt erreicht wird.

Als mögliche Salze der Verbindungen der allgemeinen Formel I kommen vor allem Alkali-, Erdalkali- und Ammoniumsalze der Phosphatgruppe in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

Die Verbindungen der allgemeinen Formel I können basische Gruppen, insbesondere Amino-Gruppen enthalten, die mit geeigneten Säuren in Säureadditionssalze überführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

In der allgemeinen Formel I bedeutet R₁ vorzugsweise eine geradkettige C₁₀-C₁₄-Alkylgruppe, die noch durch eine C₁-C₆-Alkoxy oder eine C₁-C₆-Alkylmercaptogruppe substituiert sein kann. R₁ stellt insbesondere eine Decyl-, Undecyl-, Dodecyl-, Tridecyl- oder Tetradecylgruppe dar. Als C₁-C₆-Alkoxy-substituenten von R₁ kommen vorzugsweise die Methoxy-, Ethoxy-, Butoxy- und die Hexyloxygruppen in Frage. Ist R₁ durch einen C₁-C₆-Alkylmercaptorest substituiert, versteht man darunter insbesondere den Methylmercapto-, Ethylmercapto-, Propylmercapto-, Butylmercapto- und den Hexylmercaptorest, und n eine der Zahlen 0, 1 oder 2.

R₂ bedeutet vorzugsweise eine geradkettige C₁₀-C₁₄-Alkylgruppe, die noch durch eine C₁-C₆-Alkoxygruppe oder eine C₁-C₆-Alkylmercaptogruppe substituiert sein kann. R₂ stellt insbesondere eine Decyl-, Undecyl-, Dodecyl-, Tridecyl- oder Tetradecylgruppe dar. Als C₁-C₆-Alkoxysubstituenten von R₂ kommen vorzugsweise die Methoxy-, Ethoxy-, Propoxy-, Butoxy- und die Hexyloxygruppe in Frage.

Ist R₂ durch einen C₁-C₆-Alkylmercaptorest substituiert, versteht man darunter insbesondere den Methylmercapto-, Ethylmercapto-, Butylmercapto- und Hexylmercaptorest.

R₄ und R₅ bedeuten vorzugsweise jeweils Wasserstoff oder einer der beiden Reste ist bevorzugt eine Cyano- oder Azidogruppe oder ein Halogenatom, wie Fluor, Chlor, Brom oder Jod.

Besonders bevorzugt sind Verbindungen, in denen R₃ und R₄ ein Wasserstoffatom darstellen und R₅ gleich Cyano, Azido oder Fluor ist, bzw. R₅ gleich Wasserstoff ist und R₃/R₄ eine weitere Bindung zwischen C-2' und C-3' darstellen.

In den Basen B der allgemeinen Formel I bedeuten die Reste R₆ bzw. R₇ bevorzugt ein Wasserstoffatom, einen Methyl-, Ethyl-, Propyl oder Butylrest, oder ein Halogenatom, wie Fluor, Chlor, Brom oder Jod. Besonders bevorzugt ist für R₆ bzw. R₇ ein Wasserstoffatom, der Methyl- oder Ethylrest und ein Chlor- oder Bromatom.

Der Rest R₈ ist vorzugsweise ein Wasserstoffatom, ein Methyl-, Ethyl-, Propyl- oder Butylrest, eine Aminogruppe oder ein Halogenatom wie Fluor, Chlor, Brom oder Jod, bevorzugt Chlor oder Brom.

R₁₀ bedeutet bevorzugt ein Wasserstoff-, Fluor-, Chlor- oder Bromatom, eine C₁-C₆-Alkoxygruppe, insbesondere eine Methoxy-, Ethoxy-, Propoxy-, Butoxy- oder Hexyloxygruppe, eine C₁-C₆-Alkylmercaptogruppe, insbesondere eine Methylmercapto-, Ethylmercapto-, Butylmercapto- oder Hexylmercaptogruppe, oder eine Aminogruppe, die mono- oder disubstituiert sein kann durch eine C₁-C₆-Alkylgruppe, wie z. B. die Methyl-, Ethyl-, Butyl- oder Hexylgruppe, durch eine Hydroxy-C₂-C₆-Alkylgruppe, wie z. B. die Hydroxyethyl-, Hydroxypropyl-, Hydroxybutyl- oder Hydroxyhexylgruppe, durch einen C₃-C₆-Cycloalkylrest, wie z. B. den Cyclopropyl-, Cyclopentyl- oder Cyclohexylrest, durch Aryl bevorzugt Phenyl, durch einen Aralkylrest, wie insbesondere Benzyl, das gegebenenfalls noch durch eine oder mehrere Hydroxy- oder Methoxygruppen, durch C₁-C₆-Alkylgruppen, wie z. B. die Methyl-, Ethyl-, Propyl-, Butyl- oder Hexylgruppe oder durch Halogenatome wie Fluor, Chlor oder Brom substituiert sein kann. Die Aminogruppe kann auch durch einen Hetarylalkyl- oder Hetarylrest, wie insbesondere z. B. den Thienyl-, den Furyl- oder den Pyridylrest substituiert sein. Unter dem Hetarylalkylrest versteht man bevorzugt den Thienylmethyl-, Furylmethyl- oder Pyridylmethylrest.

Bevorzugte gekoppelte Nucleoside in den beanspruchten Liponucleotiden der allgemeinen Formel I sind:
-2',3'Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2',3'-didehydro-N⁶-(o-methylbenzyl)adenosin
-2',3'-Didesoxy-2',3'-didehydro-N⁶-(2-methylpropyl)adenosin
-2',3'-Didesoxy-3'-azidoguanosin
-2',3'-Didesoxy-3'-fluor-5-chloruridin
-3'-Desoxy-3'-fluorthymidin
-2',3'-Didesoxy-3'-fluoradenosin
-2',3'-Didesoxy-2',3'-didehydrocytidin
-3'-Desoxy-2',3'-didehydrothymidin
-3'-Desoxy-3'-azidothymidin
-2',3'-Didesoxy-3'-fluor-2,6-diaminopuriuribosid
Die Verbindungen der allgemeinen Formel I können dargestellt werden, in dem man
1. eine Verbindung der allgemeinen Formel II, 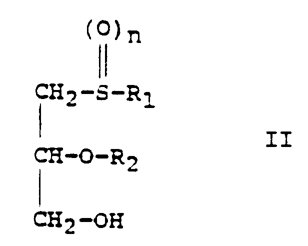 in der R₁, R₂ und n die angegebenen Bedeutungen besitzen, mit einer Verbindung der allgemeinen Formel III, 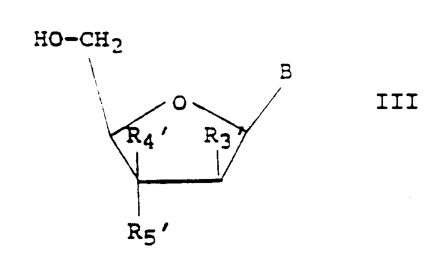 in der
   - R₃': Wasserstoff oder eine durch eine dem Fachmann geläufige Sauerstoffschutzgruppe geschützte Hydroxygruppe darstellt und
   - R₄': u. R₅' jeweils Wasserstoff, Halogen, eine Azido-, eine Cyano- oder einer der Reste R₄' und R₅' eine durch eine dem Fachmann geläufige Sauerstoffschutzgruppe geschützte Hydroxygruppe bedeutet, oder R₃' und R₄' eine weitere Bindung darstellen und
   - B: die angegebenen Bedeutungen besitzt,
   in Gegenwart von Phosphoroxitrichlorid und einem Phosphorsäureester und einer tert. Stickstoffbase, z. B. Pyridin oder Triethylamin, in einem inerten Lösungsmittel, wie z. B. Toluol zur Reaktion bringt und nach erfolgter Hydrolyse gegebenenfalls entsprechend den in der Nucleosidchemie üblichen Verfahren die Sauerstoffschutzgruppen abspaltet, oder
2. eine Verbindung der allgemeinen Formel IV, 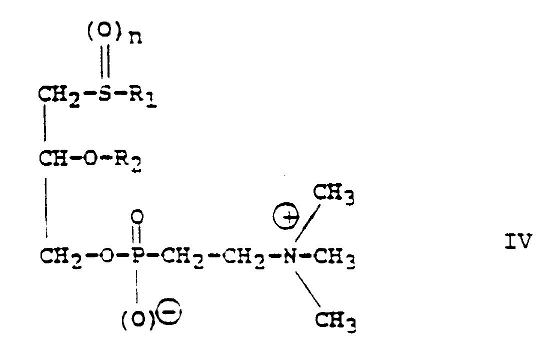 in der R₁, R₂ und n die oben genannten Bedeutungen besitzen, mit einer Verbindung der allgemeinen Formel III, in der R₃', R₄', R₅' und B die angegebenen Bedeutungen besitzen, in Gegenwart von Phospholipase D in einem inerten Lösungsmittel, wie z. B. Chloroform, in Gegenwart eines geeigneten Puffers zur Reaktion bringt und nach erfolgter Reaktion gegebenenfalls entsprechend den in der Nucleosidchemie üblichen Verfahren die Sauerstoffschutzgruppe abspaltet.

Die Herstellung der Verbindungen der allgemeinen Formel II und IV sind in Lipids 22, 947 (1987) und in der DE-A-3039629 beschrieben.

Die Herstellung der Verbindungen der allgemeinen Formel III sind beschrieben z. B. in der EP-A 0 286 028 und WO 90/08147.

Der allgemeinen Formel I ähnliche Verbindungen sind beschrieben in EP-A-0350287. Dort sind jedoch nur 1,2-Diester des Glycerins beschrieben.

Die Arzneimittel enthaltend Verbindungen der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylendiamintetraessigsäure und deren nichttoxischen Salze, hochmole-kulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage:
1. (2',3'-Didesoxy-3'-fluor-5-chloruridin)-5'-phosphorsäure-(3-dodecylmercapto-2-decyloxy)propylester
2. (3'-Desoxy-3'-azido-thymidin)-5'-phosphorsäure-(3-dodecyl-sulfinyl-2-decyloxy)propylester
3. (3'-Desoxy-3'-azido-thymidin)-5'-phosphorsäure-(3-dodecyl-sulfonyl-2-decyloxy)propylester
4. (2',3'-Didesoxycytidin)-5'-phosphorsäure-(3-dodecylmercapto-2-decyloxy)propylester
5. (2',3'-Didesoxyinosin)-5'-phosphorsäure-(3-dodecylmercapto-2-decyloxy)propylester
6. (2',3'-Didesoxyguanosin)-5'-phosphorsäure-(3-dodecylmercapto-2-decyloxy)propylester
7. (2',3'-Didesoxyadenosin)-5'-phosphorsäure-(3-dodecylmercapto-2-decyloxy)propylester
8. (3'-Desoxythymidin)-5'-phosphorsäure-(3-dodecylmercapto-2-decyloxy)propylester
9. (3'-Desoxy-2',3'-didehydrothymidin)-5'-phosphorsäure-(3-dodecylmercapto-2-decyloxy)propylester
10. (3'-Desoxy-3'-fluorthymidin)-5'-phosphorsäure-(3-dodecylmercapto-2-decyloxy)propylester
11. (2',3'-Didesoxy-3'-azidoguanosin)-5'-phosphorsäure-(3-dodecylmercapto-2-decyloxy)propylester
12. (2',3'-Didesoxy-3'-fluor-2,6-diaminopurinribosid)-5'-phosphorsäure-(3-dodecylmercapto-2-decyloxy)propylester
13. [2',3'-Didesoxy-2',3'-didehydro-N⁶-(2-methylpropyl)-adenosin]-5'-phosphorsäure-(3-dodecylmercapto-2-decyloxy)propylester
14. [2',3'-Didesoxy-2',3'-didehydro-N⁶-(o-methylbenzyl)-adenosin]-5'-phosphorsäure-(3-dodecylmercapto-2-decyloxy)propylester
15. (2',3'-Didesoxy-2',3'-didehydrocytidin)-5'-phosphorsäure-(3-decylmercapto-2-dodecyloxy)propylester
16. (2',3'-Didesoxy-3'-fluoradenosin)-5'-phosphorsäure-(3-undecylmercapto-2-dodecyloxy)propylester
17. (2',3'-Didesoxy-3'-azidouridin)-5'-phosphoräsure-(3-decylsulfonyl-2-dodecyloxy)propylester
18. (2',3'-Didesoxycytidin)-5'-phosphoräsure-(3-decylmercapto-2-decyloxy)propylester
19. (2',3'-Didesoxyinosin)-5'-phosphorsäure-(3-dodecylmercapto-2-dodecyloxy)propylester
20. (3'-Desoxy-3'-azidothymidin)-5'-phosphorsäure-(3-tetradecylmercapto-2-decyloxy)propylester
21. (3'-Desoxy-3'-azidothymidin)-5'-phosphorsäure-(3-pentadecylmercapto-2-decyloxy)propylester
22. (2',3'-Didesoxyinosin)-5'-phosphorsäure-(3-tridecylmercapto-2-decyloxy)propylester
23. (2',3'-Didesoxyinosin)-5'-phosphorsäure-(3-dodecylmercapto-2-octyloxy)propylester

### Beispiel 1 a

### (3'-Desoxy-3'-azido-thymidin)-5'-phosphorsäure-(3-dodecylmercapto-2-decyloxy)propylester

Zu einer Lösung aus 1.25 g (3 mmol) 3-Dodecylmercapto-2-decyloxy-1-propanol und 1.2 ml (8.6 mmol) Triethylamin in 40 ml abs. Ether wurden unter Stickstoff bei 0°C 0.42 ml (4.5 mmol) POCl₃ getropft und 45 min nachgerührt. Dann ließ man auf RT erwärmen, tropfte eine Lösung aus 800 mg (3 mmol) 3'-Desoxy-3'-azido-thymidin (AZT) in einer Mischung aus 15 ml abs. Ether und 20 ml abs. Toluol hinzu und rührte 6 h unter Rückfluß (DC-Kontrolle).

Nach dem Kühlen wurden 50 ml Wasser zugegeben, die Mischung für 2 h kräftig gerührt, die organische Phase danach abgetrennt, über Na₂SO₄ getrocknet und im Rotationsverdampfer eingeengt. Der Rückstand wurde durch präparative Säulenchromatographie an Kieselgel 60 mit Dichlormethan/Methanol 9:1 als Eluens gereinigt. Ausbeute 540 mg (24 % d. Th.). Schmp. 187°C Sintern, 220-223°C Zers. unter Braunfärbung, ³¹P-NMR: = 0.59 ppm.

### Beispiel 1 b

### (3'-Desoxy-3'-azido-thymidin)-5'-phosphorsäure-(3-dodecylmercapto-2-decyloxy)propylester

Analog zur Vorschrift in Chem. Pharm. Bull. 36, 5020 (1988), wurden 2 mmol AZT und 5000 U Phospholipase D in 4 ml Natriumacetatpuffer/CaCl₂ suspendiert, mit einer Lösung aus 6 mmol 3-Dodecylmercapto-2-decyloxypropyl-1-phosphorsäuremonocholinester in 160 ml Chloroform versetzt und 8 h auf 45°C erhitzt. Dann wurde über Na₂SO₄ getrocknet und das Lösungsmittel im Vakuum entfernt. Der Rückstand wurde wie in Bsp. 1 durch Säulenchromatographie gereinigt. Ausb. 51 %. Das Produkt erwies sich als identisch mit dem Produkt von Bsp. 1 a (Schmp., DC, ¹H- und ³¹ P-NMR).

### Beispiel 2

(3'-Desoxy-3'-azido-thymidin)-5'-phosphorsäure-(3-undecylmercapto-2-undecyloxy)propylester wurde in Analogie zu Bsp. 1 a hergestellt. Ausb. 27 % , Schmp. 218-222^{o} C (Zers.)

### Beispiel 3

### [2',3'-Didesoxy-2',3'-didehydro-N⁶-(o-methylbenzyl)adenosin-5'-phosphorsäure-(3-dodecylmercapto-2-decyloxy)propylester

680 mg (1.37 mmol) Phosphorsäure-(3-dodecylmercapto-2-decyloxy)propylester in 20 ml abs. Pyridin wurden mit 337 mq (1 mmol) 2',3'-Didesoxy-2',3'-didehydro-N⁶-(o-methylbenzyl) adenosin versetzt und nach Zugabe von 1.37 g (6.7 mmol) DCC 24 Stunden bei Raumtemperatur gerührt (DC-Kontrolle). Dann wurde das Pyridin im Vakuum entfernt, der Rückstand in Ether suspendiert und vom ungelösten Harnstoff abfiltriert. Das Filtrat wurde nach dem Abdampfen des Lösungsmittels durch Säulenchromatographie an Kieselgel 60 mit Dichlormethan/Methanol 95/5 als Eluens gereinigt. Ausb. 220 mg (26 % d. Th.). R_{f} = 0.68 (CH₂Cl₂/CH₃OH/H₂O 13/5/0.8).

### Beispiel 4

### (3'-Desoxy-3'-azidothymidin)-5'-phosphorsäure-(3-dodecylmercapto-2-decyloxy)propylester

Analog zu Bsp. 3 wurde aus 13.5 g Phosphorsäure-(3-dodecylmercapto-2-decyloxy)propylester, 5.4 g AZT und 27 g DCC in 350 ml abs. Pyridin durch 30-stündiges Rühren bei Raumtemperatur und Reinigung wie oben beschreiben das entsprechende Liponucleotid in 62 % Ausbeute hergestellt (Analytische Daten identisch mit denen von Bsp. 1).

### Beispiel 5

### (3'-Desoxythymidin)-5'-phosphorsäure-(3-dodecylmercapto-2-decyloxy)propylester

Analog zu Bsp. 3 wurde aus 1.3 g Phosphorsäure-(3-dodecylmercapto-2-decyloxy)propylester, 500 mg 3'-Desoxythymidin und 2.6 g DCC in 40 ml abs. Pyridin durch 24-stündiges Rühren bei Raumtemperatur und chromatographischer Reinigung das entsprechende Liponucleotid in 51 % Ausbeute erhalten. R_{f} = 0.45 (CH₂Cl₂/CH₃OH/H₂O 12/5/0.8).

### Beispiel 6

### (2',3'-Didesoxyinosin)-5'-phosphorsäure-(3-dodecylmercapto-2-decyloxy)propylester

Analog zu Bsp. 3 wurden aus 1.3 g Phosphorsäure-(3-dodecylmercapto-2-decyloxy)propylester, 500 mg 2',3'-Didesoxyinosin und 2.6 g DCC in 40 ml abs. Pyridin durch 40-stündiges Rühren bei Raumtemperatur und chromatographische Reinigung das genannte Liponucleotid in 61 % Ausbeute hergestellt. R_{f} = 0.38 (CH₂Cl₂/CH₃OH/H₂O 13/5/0.8).

## Patentansprüche

1. Verbindungen der allgemeinen Formel I 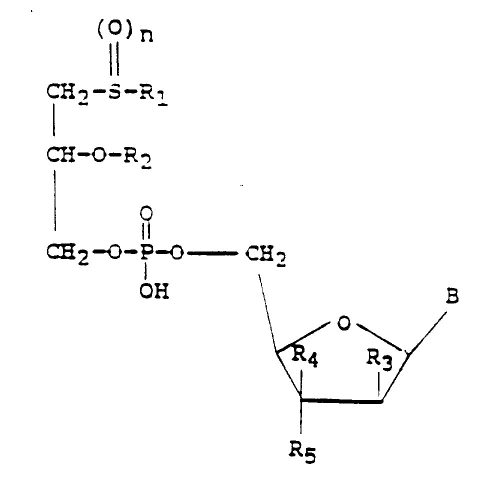 in der
R₁ eine geradkettige oder verzweigte, gesättigte oder ungesättigte Alkylkette mit 8-15 Kohlenstoffatomen, die gegebenenfalls ein oder mehrfach durch Phenyl-, Halogen, C₁-C₆-Alkoxy-, C₁-C₆-Alkylmercapto-, C₁-C₆-Alkoxycarbonyl-, C₁-C₆-Alkylsulfinyl- oder C₁-C₆-Alkylsulfonylgruppen substituiert sein kann,
R₂ eine geradkettige oder verzweigte, gesättigte oder ungesättigte Alkylkette mit 8-15 Kohlenstoffatomen, die gegebenenfalls ein oder mehrfach durch Phenyl-, Halogen, C₁-C₆-Alkoxy-, C₁-C₆-Alkylmercapto-, C₁-C₆-Alkoxycarbonyl- oder C₁-C₆-Alkylsulfonylgruppen substituiert sein kann,
R₃ Wasserstoff oder eine Hydroxygruppe
R₄, R₅ jeweils Wasserstoff oder einer der Reste R₄ und R₅ Halogen, eine Hydroxy-, eine Cyano- oder eine Azidogruppe bedeuten und außerdem R₃ und R₄ eine weitere Bindung zwischen C-2' und C-3' darstellen können,
n 0, 1 oder 2 und
B eine der folgenden Verbindungen bedeutet: 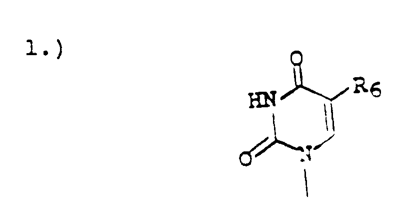
wobei R₆ Wasserstoff, eine Alkylkette mit 1-4 Kohlenstoffatomen oder Halogen sein kann, 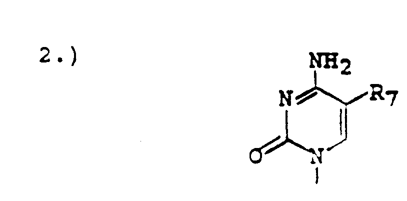 wobei R₇ Wasserstoff, eine Alkylkette mit 1-4 Kohlenstoffatomen oder Halogen sein kann, 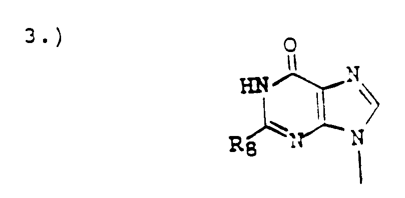 wobei R₈ Wasserstoff, eine Alkylkette mit 1-4 Kohlenstoffatomen, Halogen, oder eine Hydroxy- oder eine Aminogruppe sein kann, 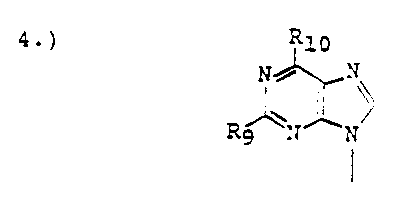 wobei R₉ Wasserstoff oder eine Aminogruppe sein kann, und R₁₀ Wasserstoff, Halogen, C₁-C₆-Alkoxy, C₁-C₆-Alkylmercapto, oder eine Aminogruppe, die mono- oder disubstituiert sein kann durch C₁-C₆-Alkyl-, C₁-C₆-Alkoxy-, Hydroxy-C₂-C₆-alkyl- und/oder C₃-C₆-Cycloalkyl-, Aryl-, Hetaryl-, Aralkyl- oder Hetarylalkylgruppen, die gegebenenfalls im Aryl- oder Hetarylrest noch durch eine oder mehrere Hydroxy-, Methoxy- oder Alkylgruppen oder Halogen substituiert sein können, oder Allyl, das gegebenenfalls mit Mono- oder Dialkyl- oder Alkoxygruppen substituiert sein kann, bedeutet,
deren Tautomere und deren physiologisch verträgliche Salze anorganischer und organischer Säuren bzw. Basen.

2. Verbindungen gemäß Anspruch 1, in der R₁ C₁₀-C₁₄-Alkyl bedeutet.

3. Verbindungen gemäß Anspruch 1 oder 2, in der R₂ C₈-C₁₂-Alkyl bedeutet.

4. Verbindungen gemäß einem der Ansprüche 1-3, in der die Gruppe 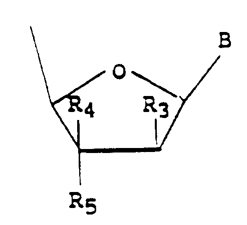 einen Rest darstellt, ausgewählt aus:
-2',3'Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2',3'-didehydro-N⁶-(o-methylbenzyl)-adenosin
-2',3'-Didesoxy-2',3'-didehydro-N⁶-(2-methylpropyl) adenosin
-2',3'-Didesoxy-3'-azidoguanosin
-2',3'-Didesoxy-3'-fluor-5-chloruridin
-3'-Desoxy-3'-fluorthymidin
-2',3'-Didesoxy-3'-fluoradenosin
-2',3'-Didesoxy-2',3'-didehydrocytidin
-3'-Desoxy-2',3'-didehydrothymidin.
-3'-Desoxy-3'-azidothymidin
-2',3'-Didesoxy-3'-fluor-2,6-diaminopurinribosid

5. Verbindungen gemäß Anspruch 1, in der R₁ Dodecyl, R₂ Decyl und die Gruppe 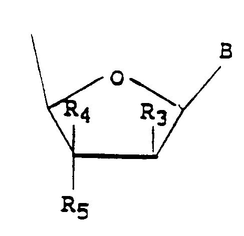 einen 2',3'-Didesoxy-2',3'-didehydro-N⁶-(0-methylbenzyl)adenosin-, 3'-Desoxy-3'-azidothymidin-, 3'-Desoxythymidin- oder 2',3'-Didesoxyinosin-Rest darstellt.

6. Verbindungen der Formel I gemäß Anspruch 1 ausgewählt aus der Gruppe bestehend aus
(3'-Desoxy-3'-azido-thymidin)-5'-phosphorsäure-(3-dodecylmercapto-2-decyloxy)propylester
(3'-Desoxy-3'-azido-thymidin)-5'-phosphorsäure-(3-undecylmercapto-2-undecyloxy)propylester
[2', 3'-Didesoxy-2',3'-didehydro-N⁶-(o-methylbenzyl)adenosin-5'-phosphorsäure-(3-dodecylmercapto-2-decyloxy)propylester
(3'-Desoxythymidin)-5'-phosphorsäure-(3-dodecylmercapto-2-decyloxy)propylester
(2',3'-Didesoxyinosin)-5'-phosphorsäure-(3-dodecylmercapto-2-decyloxy)propylester
(3'-Desoxy-2',3'-didehydrothymidin)-5'-phosphorsäure-(3-dodecylmercapto-2-decyloxy)propylester
(3'-Desoxy-3'-fluorthymidin)-5'-phosphorsäure-(3-dodecylmercapto-2-decyloxy)propylester

7. Verfahren zur Herstellung von Verbindungen der allgemeinen Formel I gemäß einem der Ansprüche 1-6,
dadurch gekennzeichnet, daß man in an sich bekannter Weise
1. eine Verbindung der allgemeinen Formel II, 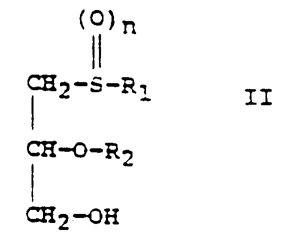 in der R₁, R₂ und n die angegebenen Bedeutungen besitzen, mit einer Verbindung der allgemeinen Formel III, 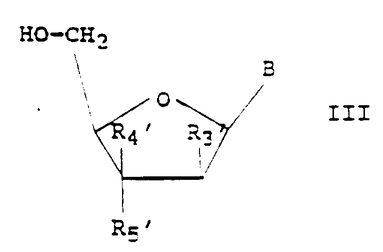 in der
R₃' Wasserstoff oder eine durch eine dem Fachmann geläufige Sauerstoffschutzgruppe geschützte Hydroxygruppe darstellt und
R₄' u. R₅' jeweils Wasserstoff, Halogen, eine Azido-, eine Cyano- oder einer der Reste R₄' und R₅' eine durch eine dem Fachmann geläufige Sauerstoffschutzgruppe geschützte Hydroxygruppe bedeutet, oder R₃' und R₄' eine weitere Bindung darstellen und
B die angegebenen Bedeutungen besitzt
in Gegenwart von Phosphoroxitrichlorid und einem Phosphorsäureester und einer tert. Stickstoffbase, z. B. Pyridin oder Triethylamin, in einem inerten Lösungsmittel, wie z. B. Toluol zur Reaktion bringt und nach erfolgter Hydrolyse gegebenenfalls entsprechend den in der Nucleosidchemie üblichen Verfahren die Sauerstoffschutzgruppen abspaltet, oder
2. eine Verbindung der allgemeinen Formel IV, 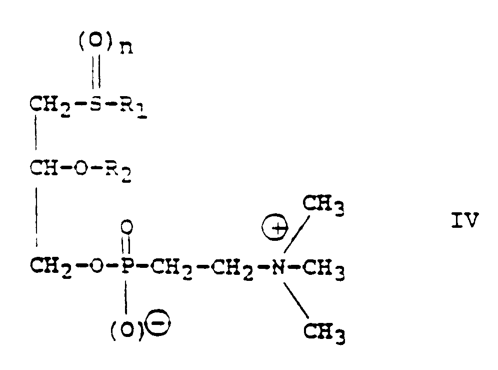 in der R₁, R₂ und n die oben genannten Bedeutungen besitzen, mit einer Verbindung der allgemeinen Formel III, in der R₃', R₄', R₅' und B die angegebenen Bedeutungen besitzen, in Gegenwart von Phospholipase D in einem inerten Lösungsmittel, wie z. B. Chloroform, in Gegenwart eines geeigneten Ruffers zur Reaktion bringt und nach erfolgter Reaktion gegebenenfalls entsprechend den in der Nucleosidchemie üblichen Verfahren die Sauerstoffschutsgruppe abspaltet,
und anschließend gewünschtenfalls die erhaltenen Verbindungen in physiologisch verträgliche Salze überführt.

8. Arzneimittel, enthaltend mindestens eine Verbindung gemäß einem der Ansprüche 1-6 und übliche Träger- und Hilfsstoffe.

9. Verwendung von Verbindungen gemäß einem der Ansprüche 1-6 zur Herstellung von Arzneimitteln zur Therapie von viralen Infektionen.

10. Verwendung von Verbindungen gemäß einem der Ansprüche 1-6 zur Herstellung von Arzneimitteln zur Behandlung der retroviralen HIV-Infektion.

## Claims

1. Compounds of the general formula I 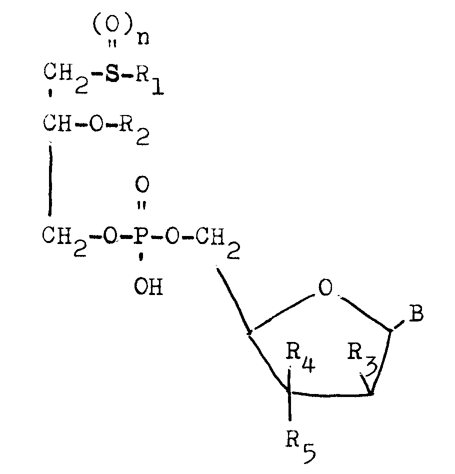 in which R₁ signifies a straight-chained or branched, saturated or unsaturated alkyl chain with 8 - 15 carbon atoms which can possibly be substituted one or more times by phenyl, halogen, C₁-C₆-alkoxy, C₁-C₆-alkylmercapto, C₁-C₆-alkoxycarbonyl, C₁-C₆-alkylsulphinyl or C₁-C₆-alkylsulphonyl groups, R₂ a straight-chained or branched, saturated or unsaturated alkyl chain with 8 - 15 carbon atoms which can possibly be substituted one or more times by phenyl, halogen, C₁-C₆-alkoxy, C₁-C₆-alkylmercapto, C₁-C₆-alkoxycarbonyl or C₁-C₆-alkylsulphonyl groups, R₃ hydrogen or a hydroxyl group, R₄, R₅ in each case hydrogen or one of the substituents R₄ and R₅ halogen, a hydroxyl, a cyano or an azido group and, furthermore, R₃ and R₄ can represent a further bond between C-2' and C-3', n O, 1 or 2 and B one of the following compounds: 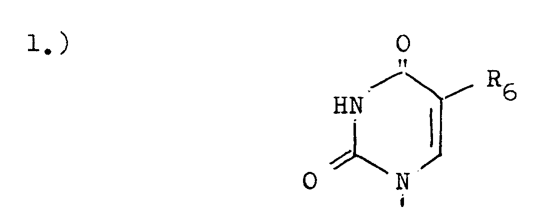 whereby R₆ can be hydrogen, an alkyl chain with 1 - 4 carbon atoms or halogen, 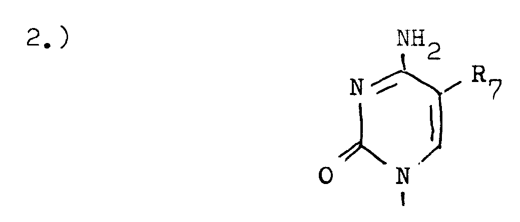 whereby R₇ can be hydrogen, an alkyl chain with 1 - 4 carbon atoms or halogen, 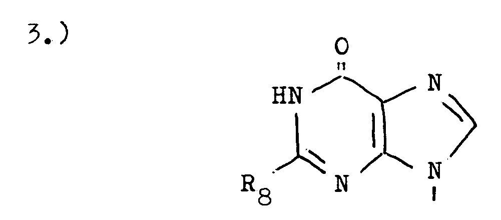 whereby R₈ can be hydrogen, an alkyl chain with 1 - 4 carbon atoms, halogen or a hydroxyl or an amino group, 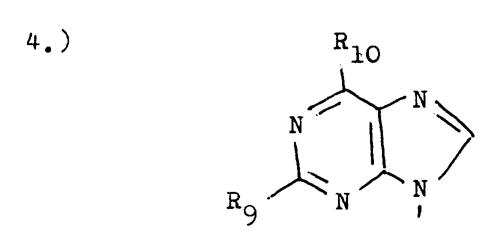 whereby R₉ can be hydrogen or an amino group and R₁₀ signifies hydrogen, halogen, C₁-C₆-alkoxy, C₁-C₆-alkylmercapto or an amino group which can be mono-or disubstituted by C₁-C₆-alkyl, C₁-C₆-alkoxy, hydroxy-C₂-C₆-alkyl and/or C₃-C₆-cycloalkyl, aryl, hetaryl, aralkyl or hetarylalkyl groups which can possibly also be substituted in the aryl or hetaryl radical by one or more hydroxyl, methoxy or alkyl groups or halogen, or allyl which can possibly be substituted with mono- or dialkyl or alkoxy groups, their tautomers and their physiologically compatible salts of inorganic or organic acids and bases.

2. Compounds according to claim 1, wherein R₁ signifies C₁₀-C₁₄-alkyl.

3. Compounds according to claim 1 or 2, wherein R₂ signifies C₈-C₁₂-alkyl.

4. Compounds according to one of claims 1 - 3, in which the group 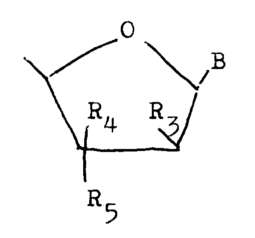 represents a radical selected from:
-2',3'-didesoxy-3'-azidouridine
-2',3'-didesoxyinosine
-2',3'-didesoxyguanosine
-2',3'-didesoxyadenosine
-3'-desoxythymidine
-2',3'-didesoxy-2',3'-didehydro-N⁶-(o-methylbenzyl)-adenosine
-2',3'-didesoxy-2',3'-didehydro-N⁶-(2-methylpropyl)-adenosine
-2',3'-didesoxy-3'-azidoguanosine
-2',3'-didesoxy-3'-fluoro-5-chlorouridine
-3'-desoxy-3'-fluorothymidine
-2',3'-didesoxy-3'-fluoroadenosine
-2',3'-didesoxy-2',3'-didehydrocytidine
-3'-desoxy-2',3'-didehydrothymidine
-3'-desoxy-3'-azidothymidine
-2',3'-didesoxy-3'-fluoro-2,6-diaminopurine riboside

5. Compounds according to claim 1, in which R₁ represents dodecyl, R₂ decyl and the group 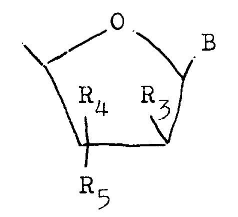 a 2',3'-didesoxy-2',3'-didehydro-N⁶-(o-methylbenzyl)-adenosine, 3'-desoxy-3'-azidothymidine, 3'-desoxythymidine or 2',3'-didesoxyinosine radical.

6. Compounds of the formula I according to claim 1 selected from the group consisting of
(3'-desoxy-3'-azidothymidine)-5'-phosphoric acid (3-dodecylmercapto-2-decyloxy)-propyl ester
(3'-desoxy-3'-azidothymidine)-5'-phosphoric acid (3-undecylmercapto-2-undecyloxy)-propyl ester
/2̅',3'-didesoxy-2',3'-didehydro-N⁶-(o-methylbenzyl)-adenosine-5'-phosphoric acid (3-dodecylmercapto-2-decyloxy)-propyl ester
(3'-desoxythymidine)-5'-phosphoric acid (3-dodecylmercapto-2-decyloxy)-propyl ester
(2',3'-didesoxyinosine)-5'-phosphoric acid (3-dodecylmercapto-2-decyloxy)-propyl ester
(3'-desoxy-2',3'-didehydrothymidine)-5'-phosphoric acid (3-dodecylmercapto-2-decyloxy)-propyl ester
(3'-desoxy-3'-fluorothymidine)-5'-phosphoric acid (3-dodecylmercapto-2-decyloxy)-propyl ester.

7. Process for the preparation of compounds of general formula I according to one of claims 1 - 6, characterised in that, in per se known manner, one
1. brings to reaction a compound of the general formula II 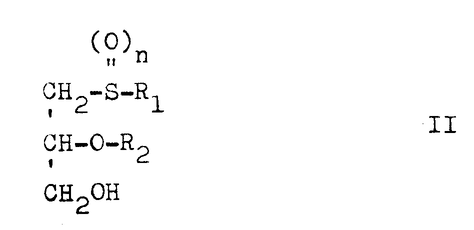 in which R₁, R₂ and n possess the given meanings, with a compound of the general formula III, 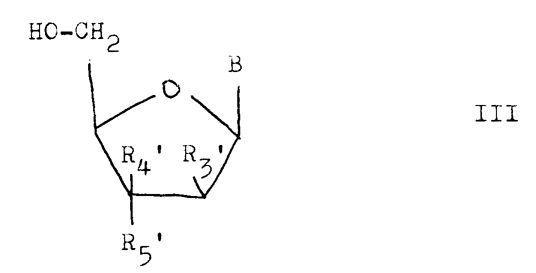 in which R₃' represents hydrogen or a hydroxyl group protected by an oxygen protective group conventional to the expert and R₄' and R₅' are each hydrogen, halogen, an azido, a cyano group or one of the radicals R₄' and R₅' signifies a hydroxyl group protected by an oxygen protective group conventional to the expert or R₃' and R₄' represent a further bond and B possesses the given meanings, in the presence of phosphorus oxychloride and aphiosphoric acid ester and of a tert. nitrogen base, e.g. pyridine or triethylamine, in an inert solvent, such as e.g. toluene, and, after hydrolysis has taken place, possibly splits off the oxygen protective groups according to processes conventional in nucleoside chemistry, or
2. brings to reaction a compound of the general formula IV 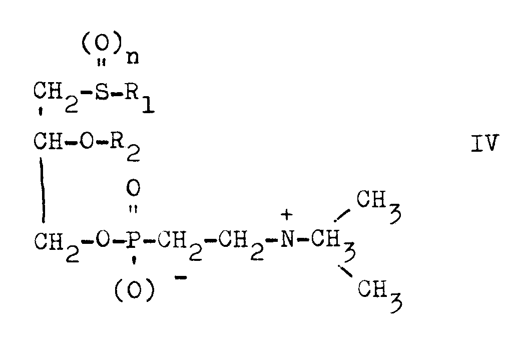 in which R₁, R₂ and n possess the above-given meanings, with a compound of the general formula III, in which R₃', R₄', R₅' and B possess the given meanings, in the presence of phospholipase D in an inert solvent, such as e.g. chloroform, and in the presence of a suitable buffer and, after the reaction has taken place, possibly splits off the oxygen protective group according to processes conventional in nucleoside chemistry; and subsequently, if desired, converts the compounds obtained into physiologically compatible salts.

8. Medicaments containing at least one compound according to one of claims 1 - 6 and usual carrier and adjuvant materials.

9. Use of compounds according to one of claims 1 - 6 for the production of medicaments for the therapy of viral infections.

10. Use of compounds according to one of claims 1 - 6 for the production of medicaments for the treatment of retroviral HIV infection.

## Revendications

1. Composés de formule générale I 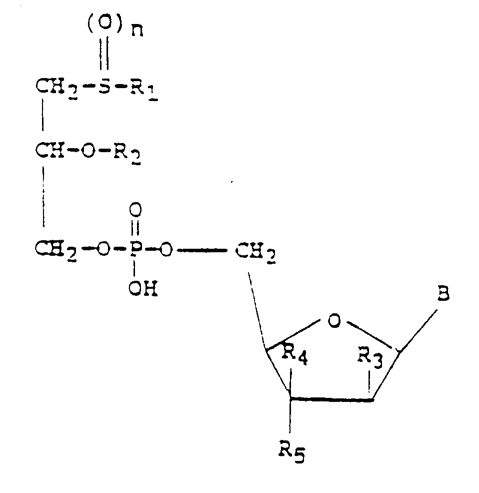 dans laquelle
R₁ représente une chaîne alkyle saturée ou insaturée, linéaire ou ramifiée, ayant 8-15 atomes de carbone, qui peut être éventuellement substituée une ou plusieurs fois par un groupe phényle, un atome d'halogène, un groupe alcoxy en C₁-C₆, alkylmercapto en C₁-C₆, alcoxycarbonyle en C₁-C₆, alkylsulfinyle en C₁-C₆ ou alkylsulfonyle en C₁-C₆,
R₂ représente une chaîne alkyle saturée ou insaturée, linéaire ou ramifiée, ayant 8-15 atomes de carbone, qui peut être éventuellement substituée une ou plusieurs fois par un groupe phényle, un atome d'halogène, un groupe alcoxy en C₁-C₆, alkylmercapto en C₁-C₆, alcoxycarbonyle en C₁-C₆ ou alkylsulfonyle en C₁-C₆,
R₃ représente un atome d'hydrogène ou un groupe hydroxy,
R₄, R₅ représentent chacun un atome d'hydrogène, ou l'un des radicaux R₄ et R₅ représente un atome d'halogène, un groupe hydroxy, cyano ou azido, et de plus, R₃ et R₄ peuvent représenter une autre liaison entre C-2' et C-3',
n vaut 0, 1 ou 2 et
B représente un des composés suivants : 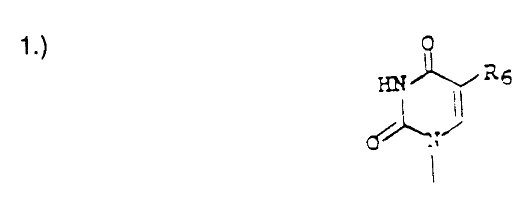
où R₆ peut être un atome d'hydrogène, une chaîne alkyle ayant 1-4 atomes de carbone ou un atome d'halogène, 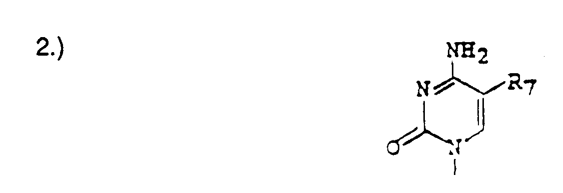 où R₇ peut être un atome d'hydrogène, une chaîne alkyle ayant 1-4 atomes de carbone ou un atome d'halogène, 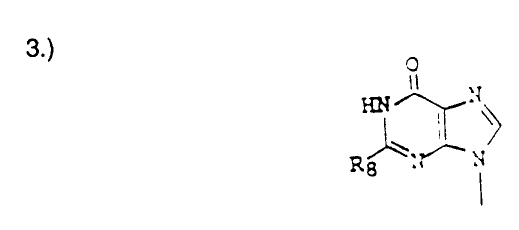 où R₈ peut être un atome d'hydrogène, une chaîne alkyle ayant 1-4 atomes de carbone, un atome d'halogène ou un groupe hydroxy ou amino, 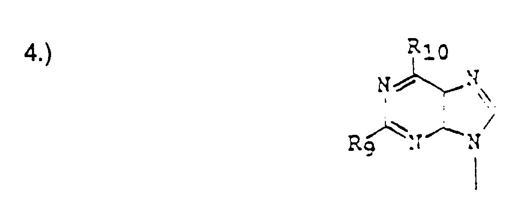 où R₉ peut être un atome d'hydrogène ou un groupe amino, et R₁₀ peut être un atome d'hydrogène, un atome d'halogène, un groupe alcoxy en C₁-C₆, alkylmercapto en C₁-C₆ ou amino, qui peut être mono- ou disubstitué par un groupe alkyle en C₁-C₆, alcoxy en C₁-C₆, hydroxyalkyle en C₂-C₆ et/ou cycloalkyle en C₃-C₆, aryle, hétéroaryle, aralkyle ou hétéroarylalkyle, dont le radical aryle ou hétéroaryle être éventuellement substitué par un ou plusieurs groupes hydroxy, méthoxy ou alkyle, ou par un atome d'halogène, ou un groupe allyle qui peut être éventuellement substitué par des groupes mono- ou dialkyle ou alcoxy,
leurs tautomères et leurs sels physiologiquement acceptables, formés avec des acides ou bases organiques ou inorganiques.

2. Composés selon la revendication 1, dans lesquels R₁ représente un groupe alkyle en C₁₀-C₁₄.

3. Composés selon la revendication 1 ou 2, dans lesquels R₂ représente un groupe alkyle en C₈-C₁₂.

4. Composés selon l'une quelconque des revendications 1-3, dans lesquels le groupe 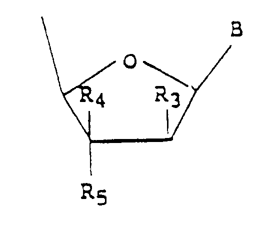 représente un radical choisi parmi les composés suivants :
2',3'-didésoxy-3'-azidouridine
2',3'-didésoxyinosine
2',3'-didésoxyguanosine
2',3'-didésoxycytidine
2',3'-didésoxyadénosine
3'-désoxythymidine
2',3'-didésoxy-2',3'-didéhydro-N⁶-(o-méthylbenzyl)-adénosine
2',3'-didésoxy-2',3'-didéhydro-N⁶-(2-méthylpropyl)-adénosine
2',3'-didésoxy-3'-azidoguanosine
2',3'-didésoxy-3'-fluoro-5-chloruridine
3'-désoxy-3'-fluorothymidine
2',3'-didésoxy-3'-fluoroadénosine
2',3'-didésoxy-2',3'-didéhydrocytidine
3'-désoxy-3'-azidothymidine
2',3'-didésoxy-3'-fluoro-2,6-diaminopurineriboside.

5. Composés selon la revendication 1, dans lesquels R₁ représente un groupe dodécyle, R₂ représente un groupe décyle et le groupe 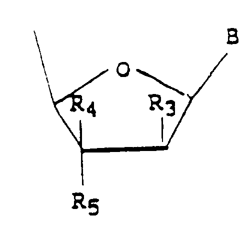 représente un groupe 2',3'-didésoxy-2',3'-didéhydro-N⁶-(o-méthylbenzyl)-adénosine, 3'-désoxy-3'-azidothymidine, 3'-désoxythymidine ou 2',3'-didésoxyinosine.

6. Composés de formule I selon la revendication 1, choisis dans le groupe formé des composés suivants ;
ester (3-dodécylmercapto-2-décyloxy)propylique de l'acide (3'-désoxy-3'-azidothymidine)-5'-phosphorique
ester (3-undécylmercapto-2-undécyloxy)propylique de l'acide (3'-désoxy-3'-azidothymidine)-5'-phosphorique
ester (3-dodécylmercapto-2-décyloxy)propylique de l'acide [2',3'-didésoxy-2',3'-didéhydro-N⁶-(o-méthylbenzyl)-adénosine]-5'-phosphorique
ester (3-dodécylmercapto-2-décyloxy)propylique de l'acide (3'-désoxythymidine)-5'-phosphorique
ester (3-dodécylmercapto-2-décyloxy)propylique de l'acide (2',3'-didésoxyinosine)-5'-phosphorique
ester (3-dodécylmercapto-2-décyloxy)propylique de l'acide (3'-désoxy-2',3'-didéhydrothymidine)-5'-phosphorique
ester (3-dodécylmercapto-2-décyloxy)propylique de l'acide (3'-désoxy-3'-fluorothymidine)-5'-phosphorique.

7. Procédé de préparation de composés de formule générale I, selon l'une quelconque des revendications 1-6, caractérisé en ce que, de manière connue en soi,
1. on fait réagir un composé de formule générale II, 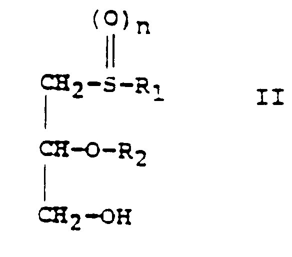 dans laquelle
R₁, R₂ et n ont les significations mentionnées plus haut, avec un composé de formule générale III 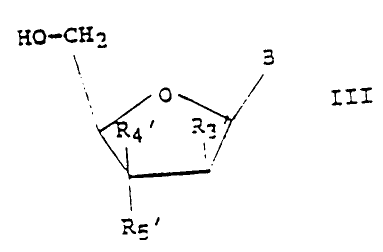
R₃' représente un atome d'hydrogène ou un groupe hydroxy protégé par un groupe protecteur d'oxygène connu par l'homme de l'art,
R₄' et R₅' représentent chacun un atome d'hydrogène, un atome d'halogène, un groupe azido, cyano, ou l'un des radicaux R₄' et R₅' représente un groupe hydroxy protégé par un groupe protecteur d'oxygène connu par l'homme de l'art, ou R₃' et R₄' représentent une autre liaison, et
B a la signification mentionnée,
en présence d'oxytrichlorure de phosphore et d'un ester de l'acide phosphorique et d'une base d'azote tertiaire, par exemple la pyridine ou la triéthylamine, dans un solvant inerte, tel que par exemple le toluène, et après le déroulement de l'hydrolyse, on sépare le groupe protecteur d'oxygène selon un procédé usuel dans la chimie des nucléosides, ou
2. on fait réagir un composé de formule générale IV, 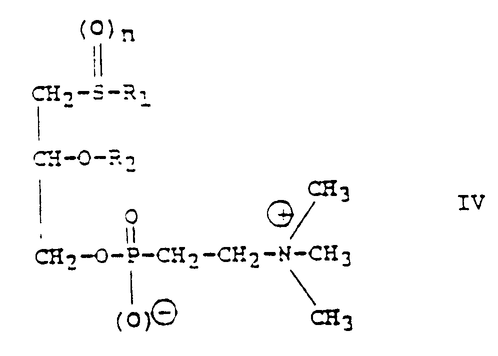 dans laquelle R₁, R₂ et n ont les significations mentionnées plus haut, avec un composé de formule générale III, dans laquelle R₃', R₄', R₅' et B ont les significations mentionnées ci-dessus, en présence de phospholipase D dans un solvant inerte, tel que par exemple le chloroforme, en présence d'un tampon approprié, et après la fin de la réaction, on sépare éventuellement le groupe protecteur d'oxygène selon un procédé usuel dans la chimie des nucléosides,
et ensuite, on transforme éventuellement les composés obtenus en leurs sels physiologiquement acceptables.

8. Médicament contenant au moins un composé selon l'une quelconque des revendications 1-6, et des véhicules et adjuvants usuels.

9. Utilisation de composés selon l'une quelconque des revendications 1-6, pour la préparation de médicaments destinés au traitement d'infections virales.

10. Utilisation de composés selon l'une quelconque des revendications 1-6, pour la préparation de médicaments destinés au traitement d'infections par le retrovirus HIV.
